# EUROPEAN PATENT APPLICATION

(11) **EP 2 520 823 A1**
(43) Date of publication of application: **07.11.2012**
(21) Application number: 10840812.1
(22) Date of filing: 30.09.2010
(51) Int. Cl.: F16F 7/12, A61F 2/28, A61F 2/30, A61L 27/00, F16F 7/00

(54) **SHOCK ABSORBING STRUCTURE AND METHOD OF MANUFACTURING SAME**

(30) Priority: 28.12.2009 JP 2009298803
(71) Applicant: Nakashima Medical Co., Ltd., Okayama-shi, Okayama 709-0625 (JP)
(72) Inventor: NAKANO, Takayoshi, Suita-shi Osaka 565-0871 (JP); KURAMOTO, Koichi, Okayama-shi Okayama 709-0625 (JP); ISHIMOTO, Takuya, Suita-shi Osaka 565-0871 (JP); IKEO, Naoko, Suita-shi Osaka 565-0817 (JP); FUKUDA, Hidetsugu, Okayama-shi Okayama 709-0625 (JP); NOYAMA, Yoshihiro, Okayama-shi Okayama 709-0625 (JP)
(74) Representative: Zimmermann & Partner
(86) International application number: PCT/JP2010/067146
(87) International publication number: WO 2011/080953

(57) **Abstract**

A shock absorbing structure having a high shock absorption characteristic is provided. The shock absorbing structure (1) includes a solidified portion (2) and a sintered portion (3). The solidified portion (2) is formed by dissolving and solidifying a plurality of inorganic powder particles. The sintered portion (3) is formed by sintering a plurality of the inorganic powder particles. The sintered portion (3) is connected to the solidified portion (2). The shock absorbing structure (1) is a composite structure including the solidified portion (2) and the sintered portion (3) and therefore has a high shock absorption characteristic.

## Description

### TECHNICAL FIELD

The present invention relates to a shock absorbing structure and a method of manufacturing the same, and more specifically to a shock absorbing structure for use in a medical implant such as an artificial joint and a bone plate and a transportation such as an automobile, an airplane, and a ship and a method of manufacturing the same.

### BACKGROUND ART

JP 2005-329179 A (Patent Document 1) and JP 6-90971 A (Patent Document 2) disclose metal implants. The metal implants disclosed by these documents consist of metals such as titanium alloy.

### SUMMARY OF THE INVENTION

### PROBLEMS TO BE SOLVED BY THE INVENTION

An implant is buried in a living body and used for a long period in the body. Therefore, the implant must have a mechanical characteristic analogous to bones. More specifically, the implant must have a shock absorption characteristic. Furthermore, the implant must have low Young's modulus and lightness approximate to those of bones.

The metal implant disclosed by Patent Document 1 however consists of a solid metal material. Therefore, the Young's modulus of the metal implant is significantly larger than that of a bone. A solid material made of a bio-compatible metal, T1-6A1-4V alloy has Young's modulus about as large as 110 GPa, while the Young's modulus of a bone (cortical bone) is about from 10 GPa to 30 GPa. Furthermore, the solid material has high yield stress and is not easily plastically deformed. If there is any plastic deformation, work hardening occurs in the solid material. Therefore, the solid material has a low shock absorption characteristic.

Meanwhile, the metal implant disclosed by Patent Document 2 has hollow inside. Therefore, it may have lower Young's modulus than that of the solid metal implant. However, even having the hollow portion, the metal implant has a low shock absorption characteristic.

Therefore, there is a demand for a new implant having a greater shock absorption characteristic than that of the conventional implants.

Such a demand for an improved shock absorption characteristic is not limited to that of the implants. For example, there is a demand for a higher shock absorption characteristic in a structure for use in a transportation such as an automobile, an airplane, a ship, and a train.

### DISCLOSURE OF THE INVENTION

An object of the present invention is to provide a shock absorbing structure having a high shock absorption characteristic.

Another object of the present invention is to provide a shock absorbing structure that has a high shock absorption characteristic, low Young's modulus, and lightness.

A shock absorbing structure according to the present invention includes a solidified portion and a sintered portion. The solidified portion is formed by dissolving a plurality of inorganic powder particles. The sintered portion is formed by sintering a plurality of the inorganic powder particles and connected to the solidified portion. Here, the sintered portion may be connected to the solidified portion by sintering or by a part of the sintered portion or solidified portion that is melted.

The shock absorbing structure according to the present invention is a composite structure including a solidified portion and a sintered portion and therefore has a high shock absorption characteristic.

The sintered portion preferably includes a plurality of necks and gaps. The plurality of necks are formed between the plurality of inorganic powder particles. The gaps are formed between the plurality of inorganic powder particles.

Since necks and gaps are formed, a stress-strain curve of the shock absorbing structure according to the present invention has a plateau region. Therefore, the shock absorbing structure has a high shock absorption characteristic. The sintered portion has the gaps and has a lower density than that of a solid material. Therefore, the sintered portion has better lightness and lower Young's modulus than those of the solid material.

The solidified portion preferably includes a solidified case. The sintered portion is stored in and connected to the solidified case.

In this way, the shock absorbing structure is lighter and has lower Young's modulus and a higher shock absorption characteristic than the solid material.

The solidified portion preferably further includes a solidified wall and a plurality of storing chambers. The solidified wall is formed in the solidified case. The plurality of storing chambers are provided in the solidified case and partitioned by the solidified wall. The shock absorbing structure further includes a plurality of sintered portions. The plurality of sintered portions are stored in the storing chambers and connected to the solidified case and/or the solidified wall.

In this way, the shock absorption characteristic improves.

Preferably, a plurality of solidified portions are sequentially layered on one another by an layered manufacturing method, so that the solidified portion that stores a plurality of the inorganic powder particles is formed, and the solidified portion thus formed is heated in a furnace at a sintering temperature less than a melting point of the inorganic powder particles, so that the sintered portion is formed.

In the shock absorbing structure according to the present invention, the solidified portion is formed by an layered manufacturing method. Therefore, the shape of the solidified portion can be set freely, and better lightness, lower Young's modulus, and a high shock absorption characteristic are obtained as compared to the solid material having the same composition. A plurality of inorganic powder particles are stored in the solidified portion shaped by the layered manufacturing method, so that the sintered portion can be easily formed in the solidified portion by sintering process.

Preferably, in the shock absorbing structure according to the present invention, a plurality of shock absorbing layers are sequentially layered by an layered manufacturing method. Each of the shock absorbing layers includes a solidified portion formed by irradiating a powder layer made of a plurality of the inorganic powder particles with a first electron beam, thereby dissolving a first region of the powder layer, and a sintered portion formed by irradiating the powder layer with a second electron beam having a fluence lower than that of the first electron beam, thereby sintering a second region of the powder layer different from the first region.

In this way, the sintered portion can be formed while the solidified portion is formed by the layered manufacturing method. Therefore, the solidified portion formed by the layered manufacturing method does not have to be sintered.

The powder particles are preferably made of a metal. The solidified portion preferably has the same composition as that of the sintered portion. The solidified portion and the sintered portion are more preferably made of titanium alloy. The shock absorbing structure even more preferably has Young's modulus from 10 GPa to 50 GPa.

In this way, the shock absorbing structure can have Young's modulus approximate to that of a bone. Therefore, the shock absorbing structure can be used as a medical implant having lightness, a shock absorption characteristic, and low Young's modulus.

A method of manufacturing a shock absorbing structure according to the present invention is a method of manufacturing the above-described shock absorbing structure and includes the steps of forming a powder layer made of a plurality of the inorganic powder particles, forming a solidified portion by irradiating the powder layer with an electron beam and dissolving the inorganic powder particles, layering a new powder layer made of the plurality of inorganic powder particles on the powder layer provided with the solidified portion, forming a new solidified portion by irradiating the new powder layer with an electron beam, forming a solidified portion made of the plurality of the solidified portions layered on one another and storing a plurality of the inorganic powder particles by repeating the layering step and the forming step, taking out the solidified portion from the powder layer, and forming the sintered portion by heating the taken out solidified portion at a sintering temperature less than a melting point of the inorganic powder particles.

By the method of manufacturing a shock absorbing structure according to the present invention, the shape of the solidified portion can be set freely. Furthermore, by controlling the design of the solidified portion and the sintering condition, a shock absorbing structure having Young's modulus and a shock absorption characteristic as desired can be manufactured.

A method of manufacturing a shock absorbing structure according to the present invention is a method of manufacturing the above-described shock absorbing structure and includes the steps of forming a powder layer made of a plurality of inorganic powder particles, forming a solidified portion by irradiating a first electron beam into the powder layer and dissolving a plurality of the powder particles, forming a sintered portion by irradiating the powder layer with a second electron beam with a fluence lower than that of the first beam and sintering a plurality of the inorganic powder particles, layering a new powder layer on the powder layer provided with the solidified portion and the sintered portion, forming the solidified portion and the sintered portion with the new powder layer, and forming the shock absorbing structure including the solidified portion made of a plurality of the solidified portions layered on one another and the sintered portion made of a plurality of the sintered portions layered on one another by repeating the layering step and the forming step.

By the method of manufacturing a shock absorbing structure according to the present invention, a shock absorbing structure having Young's modulus, lightness, and a shock absorption characteristic as desired can be manufactured by controlling the design of the solidified portion and the sintering condition.

### BRIEF DESCRIPTION OF THE DRAWINGS

Fig. 1 is a perspective view of a shock absorbing structure according to a first embodiment of the present invention.
Fig. 2 is a perspective view of a solidified portion shown in Fig. 1.
Fig. 3 is a sectional view taken along line III-III in Fig. 1.
Fig. 4 is an enlarged view of a region 500 shown in Fig. 3.
Fig. 5 is a view of a layered manufacturing machine used to manufacture the shock absorbing structure shown in Fig. 1
Fig. 6 is a flowchart for use in illustrating a method of manufacturing the shock absorbing structure shown in Fig. 1.
Fig. 7 is a schematic view for use in illustrating process in step S6 in Fig. 6.
Fig. 8 is a schematic view for use in illustrating process in step S8 shown in Fig. 6.
Fig. 9 is a schematic view for use in illustrating process in step S11 shown in Fig. 6.
Fig. 10 is a schematic view for use in illustrating process in step S6 in Fig. 6 that is repeatedly carried out, showing its second time and on.
Fig. 11 is a schematic view for use in illustrating process in step S8 in Fig. 6 that is repeatedly carried out, showing its second time and on.
Fig. 12 is a sectional view of a solidified portion in the process of manufacturing taken in the vertical direction.
Fig. 13 is a view for use in illustrating process in step S12 in Fig. 6.
Fig. 14 is a sectional view of a solidified portion manufactured by a manufacturing step in Fig. 6 taken in the vertical direction.
Fig. 15 is a SEM (Scanning Electron Microscopy) image of a sintered portion in a shock absorbing structure manufactured by the manufacturing method in Fig. 6.
Fig. 16 is another SEM image of the sintered portion in association with Fig. 15.
Fig. 17 is another SEM image of the sintered portion different from Figs. 15 and 16.
Fig. 18 is another SEM image of the sintered portion in association with Fig. 17.
Fig. 19 is a stress-strain curve of the shock absorbing structure according to the embodiment.
Fig. 20 is a stress-strain curve of the shock absorbing structure different from Fig. 19.
Fig. 21 is a stress-strain curve of a shock absorbing structure different from Figs. 19 and 20.
Fig. 22A is a perspective view of a shock absorbing structure having a different arrangement from Fig. 1.
Fig. 22B is a perspective view of a region surrounded by a dashed line in Fig. 22A.
Fig. 23A is a perspective view of the shock absorbing structure having a different arrangement from Fig. 1 and Fig. 22A.
Fig. 23B is a perspective view of a region surrounded by a dashed line in Fig. 23A.
Fig. 24 is a perspective view of a shock absorbing structure according to a second embodiment of the present invention.
Fig. 25 is a sectional view taken along line XXV-XXV in Fig. 24.
Fig. 26 is a flowchart for use in illustrating a method of manufacturing the shock absorbing structure shown in Fig. 24.
Fig. 27 is a stress-strain curve of the shock absorbing structure shown in Fig. 24.
Fig. 28 is a perspective view of the shock absorbing structure having a different arrangement from those in Figs. 1, and 22 to 25.

### BEST MODE FOR CARRYING OUT THE INVENTION

Now, an embodiment of the present invention will be described in conjunction with the accompanying drawings in which the same or corresponding portions are designated by the same reference characters and their description will not be repeated.

### First Embodiment

### Constitution of Shock Absorbing Structure

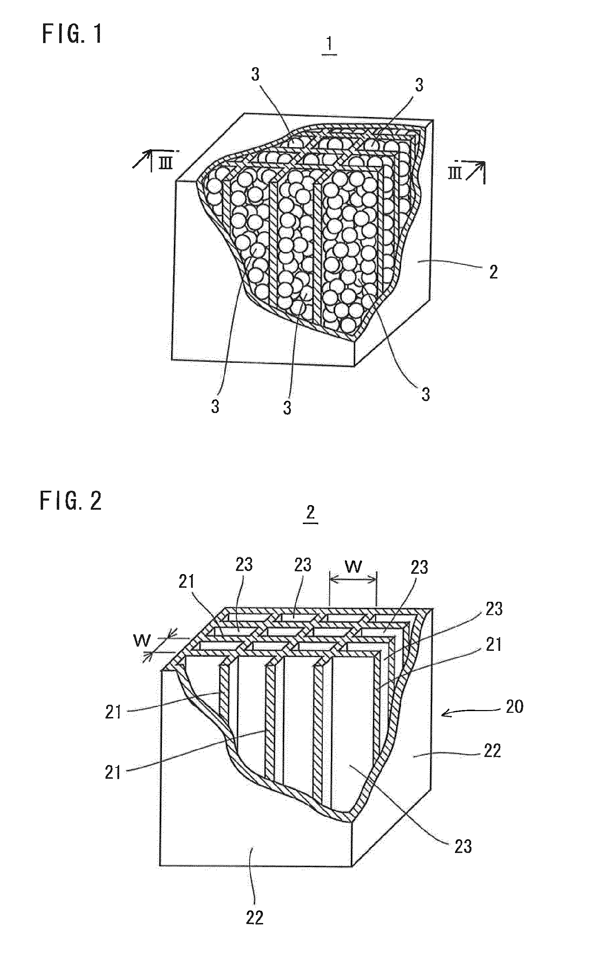

Fig. 1 is a perspective view of a shock absorbing structure according to the embodiment. Referring to Fig. 1, the shock absorbing structure 1 includes a solidified portion 2 and a plurality of sintered portions 3.

A plurality of inorganic powder particles melt and then solidify to form the solidified portion 2. The inorganic powder particles are powder particles of an inorganic substance. Examples of the inorganic powder particles include a metal, an intermetallic compound, and ceramics. The metal is for example a pure metal or an alloy. The inorganic powder particles are preferably a metal.

Fig. 2 is a perspective view of the solidified portion 2. The solidified portion 2 includes a solidified case 20 and a plurality of solidified walls 21. The solidified case 20 has a plurality of solidified walls 22. More specifically, the solidified walls 22 correspond to the outer walls of the solidified case 20. The plurality of solidified walls 21 are stored in the solidified case 20. More specifically, the solidified walls 21 correspond to inner walls that partition the inside of the solidified case 20. The solidified case 20 has a plurality of storing chambers 23 partitioned by the plurality of solidified walls 21.

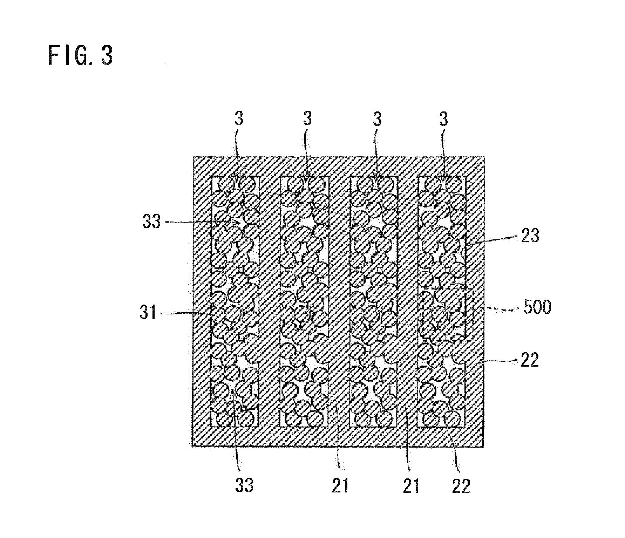

Fig. 3 is a sectional view taken along line III-III in Fig. 1. Referring to Fig. 3, in the shock absorbing structure 1, a plurality of sintered portions 3 are each stored in a storing chamber 23. A plurality of inorganic powder particles are sintered and formed into the sintered portion 3. The sintered portion 3 is made from inorganic powder particles in the same composition as that of the solidified portion 2. In short, the sintered portions 3 and the solidified portion 2 have substantially the same composition.

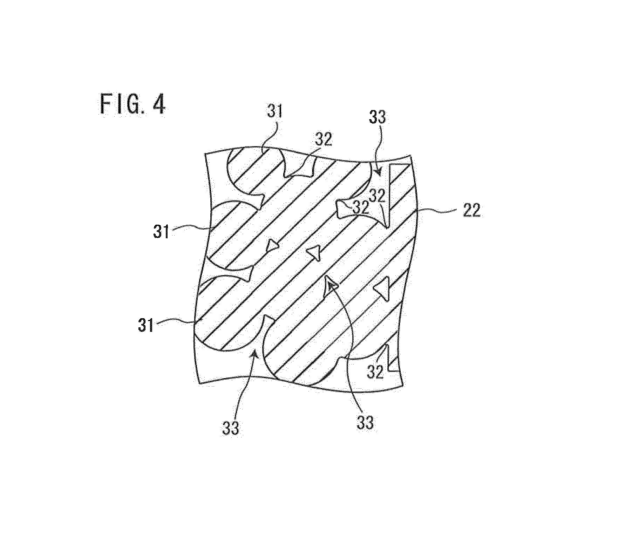

Fig. 4 is an enlarged view of a region 500 in Fig. 3. Referring to Fig. 4, the sintered portion 3 includes a plurality of inorganic powder particles 31 and a plurality of necks 32. The plurality of necks 32 are formed between the plurality of inorganic powder particles 31. During the process of sintering, some of adjacent inorganic powder particles 31 are connected by sintering to form a neck 32. The process of forming the neck 32 is called "necking."

The neck 32 is also formed between the inorganic powder particle 31 and the solidified walls 22. As shown in Figs. 3 and 4, the necks 32 connect the sintered portions 3 to the solidified walls 21 and 22 of the solidified portion 2. The necks 32 are formed by atomic diffusion.

In Figs. 3 and 4, the sintered portions 3 are connected by the necks 32. However, the sintered portions 3 may be connected by other methods. For example, the sintered portions 3 and/or solidified portion 2 may be partly melted, so that the sintered portions 3 and the solidified portion 2 are connected.

As shown in Figs. 3 and 4, the sintered portions 3 have a plurality of gaps 33. The plurality of gaps 33 are formed between the plurality of inorganic powder particles 31. The porosity of the sintered portions 3 is for example from 30% to 82%.

### Method of Manufacturing Shock Absorbing Structure

A shock absorbing structure 1 having the above-described constitution is manufactured by a rapid prototyping method, more specifically by an layered manufacturing method. In the following, an example of the method of manufacturing the shock absorbing structure 1 will be described.

### Structure of Layered manufacturing machine

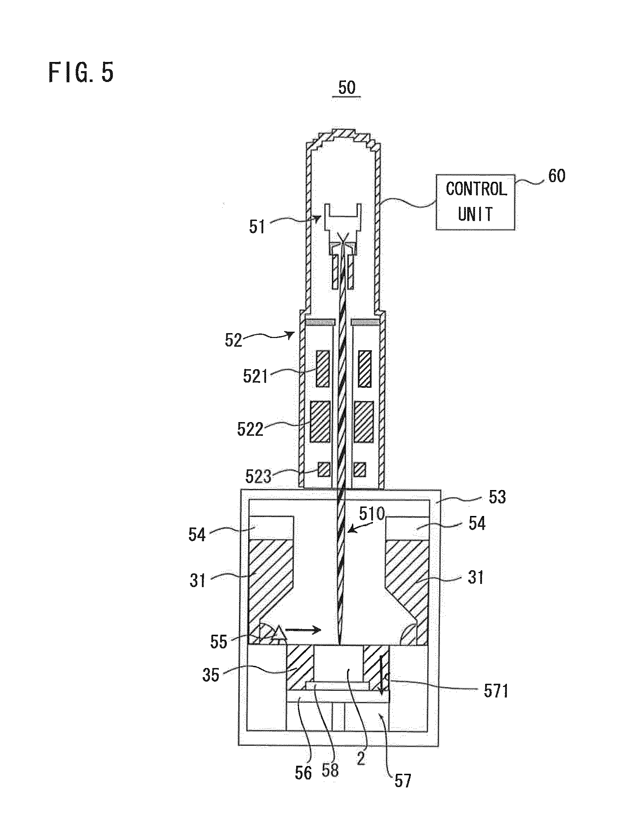

Fig. 5 is a view of a layered manufacturing machine used to manufacture the shock absorbing structure 1. Referring to Fig. 5, the layered manufacturing machine 50 includes an irradiator 51, a regulator 52, a manufacturing chamber 53, and a control unit 60.

The irradiator 51 is provided in the upper part of the layered manufacturing machine 50. The irradiator 51 irradiates an electron beam 510 downward. The regulator 52 is provided under the irradiator 51. The regulator 52 deflects the electron beam 510 in response to a command from the control unit 60. In this way, the electron beam 510 is directed upon a prescribed region. The regulator 52 further corrects the focal point or astigmatism of the electron beam 510. In this way, the fluence of the electron beam 510 (the amount of energy provided per unit area) is regulated.

The regulator 52 includes an astigmatism coil 521, a focus coil 522, and a deflecting coil 523. The astigmatism 521 corrects the astigmatism of the electron beam 510. The focus coil 522 corrects the focal point of the electron beam 510. The deflection coil 523 deflects the electron beam 510. More specifically, the deflection coil 523 changes the irradiating direction of the electron beam 510.

The manufacturing chamber 53 is provided under the regulator 52. In the manufacturing chamber 53, a solidified portion 2 is formed. The manufacturing chamber 53 is connected to a vacuum pump that is not shown. When the solidified portion 2 is manufactured, the manufacturing chamber 53 is subjected to vacuum drawing.

The manufacturing chamber 53 includes a pair of powder supply devices 54, a rake 55, a modeling table 56, a powder storing chamber 57, and a base plate 58.

The powder storing chamber 57 is provided in the center of the lower portion of the manufacturing chamber 53. The powder storing chamber 57 has a case shape having an opening on the upper end and has a side wall 571. The modeling table 56 is stored in the powder storing chamber 57 and supported so that it can be moved up and down. The modeling table 56 is elevated/lowered by a motor that is not shown. The base plate 58 is provided on the modeling table 56. The solidified portion 2 is formed on the base plate 58. The base plate 58 can prevent the solidified portion 2 from being connected onto the modeling table 56.

The pair of powder supply devices 54 is provided above the powder storing chamber 57 and has the powder storing chamber 57 therebetween when it is viewed from above the layered manufacturing machine 50. The powder supply device 54 stores a plurality of inorganic powder particles 31 as a raw material for the solidified portion 2 and the sintered portions 3, and discharges a plurality of inorganic powder particles 31 in response to a command from the control unit 60.

The rake 55 is provided near an upper end of the powder storing chamber 57. The rake 55 is moved horizontally by a motor that is not shown and reciprocates between the pair of powder supply devices 54. The horizontal movement of the rake 55 allows inorganic powder particles 31 discharged from the powder supply devices 54 to be supplied to the powder storing chamber 57. A plurality of inorganic powder particles 31 accumulated in the powder storing chamber 57 form a powder layer 35 on the modeling table 56. The rake 55 flattens the surface of the powder layer 35 as it moves horizontally.

The control unit 60 includes a central processing unit (CPU), a memory, and a hard disk drive (hereinafter as "HDD") that are not shown. The HDD stores a well known CAD (Computer Aided Design) application and a CAM (Computer Aided Manufacturing) application. The control unit 60 uses the CAD application to manufacture three-dimensional shape data for the shock absorbing structure 1.

The control unit 60 further uses the CAM application and manufactures processing condition data based on the three-dimensional data. In the layered manufacturing method, a plurality of solidified portions formed by an electron beam 510 are layered upon one another to form the solidified portion 2. The processing condition data includes processing conditions when each of the solidified portions are formed. More specifically, such processing condition data is manufactured for each of the solidified portions.

The control unit 60 controls the electron beam 510 based on each pieces of processing condition data to form a corresponding solidified portion.

### Details of Manufacturing Process

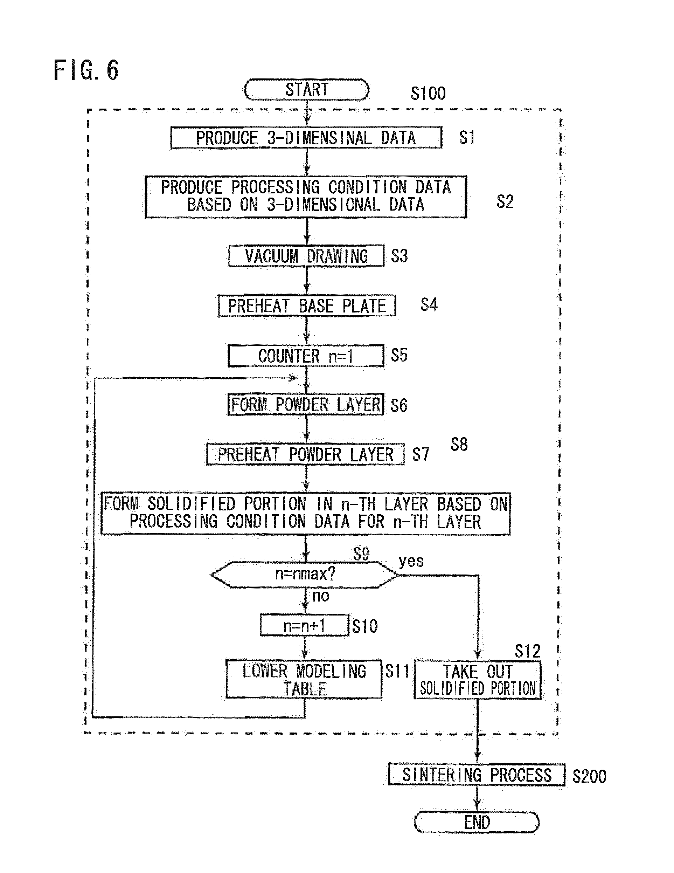

Fig. 6 is a flowchart showing details of a method of manufacturing the shock absorbing structure 1. Referring to Fig. 6, the solidified portion 2 is formed by the layered manufacturing method to start with (S100: manufacturing step). Then, sintered portions 3 are formed by sintering processing (S200: sintering step). Through these manufacturing step and sintering step, the shock absorbing structure 1 is manufactured. Now, the manufacturing process will be described in detail.

### Manufacturing step (S100)

In the manufacturing step (S100), the control unit 60 manufactures three-dimensional data for the shock absorbing structure 1 using the CAD application (S1). The manufactured three-dimensional data is stored in the memory in the control unit 60. Then, the control unit 60 uses the CAM application to manufacture processing condition data based on the three-dimensional data (S2).

As described above, the processing condition data is manufactured for each of the solidified portions. To start with, a case in which the shock absorbing structure 1 is sliced into a predetermined number of layers nmax (number). At the time, the shape of each of the plurality of solidified portions formed by slicing the solidified portion 2 is a plate shape, a frame shape, or a grid shape. Processing condition data for solidified portions in n-th layer (n: natural number from 1 to nmax) is manufactured by the following method. Here, the first layer is the lowermost layer and the nmax layer is the uppermost layer.

The control unit 60 manufactures sectional shape data for the solidified portion 2 in the n-th layer based on the three-dimensional data. The control unit 60 then manufactures processing condition data based on the sectional shape data. The processing condition data includes a region condition and a fluence condition. The control unit 60 determines a region to be irradiated with an electron beam based on the sectional shape data and defines it as a region condition. Then, based on the fluence necessary for forming the solidified portions, the current value, the scanning rate, the scanning interval value, and the electron focus value of the electron beam 510 are determined and defined as the fluence condition. Information about the fluence is stored in advance in the HDD in the control unit 60 corresponding to compositions of inorganic powder particles. Through the above-described steps, the processing condition data for each of the layers is manufactured. The plurality pieces of manufactured condition data are stored in the memory in the control unit 60.

Then, using a vacuum pump, the manufacturing chamber 53 is evacuated (S3). After the manufacturing chamber 53 is evacuated, the base plate 58 provided on the modeling table 56 is pre-heated (S4).

The control unit 60 sets counter n to "1" (S5) and starts to manufacture the solidified portion in the first layer (lowermost layer) (S6 to S8).

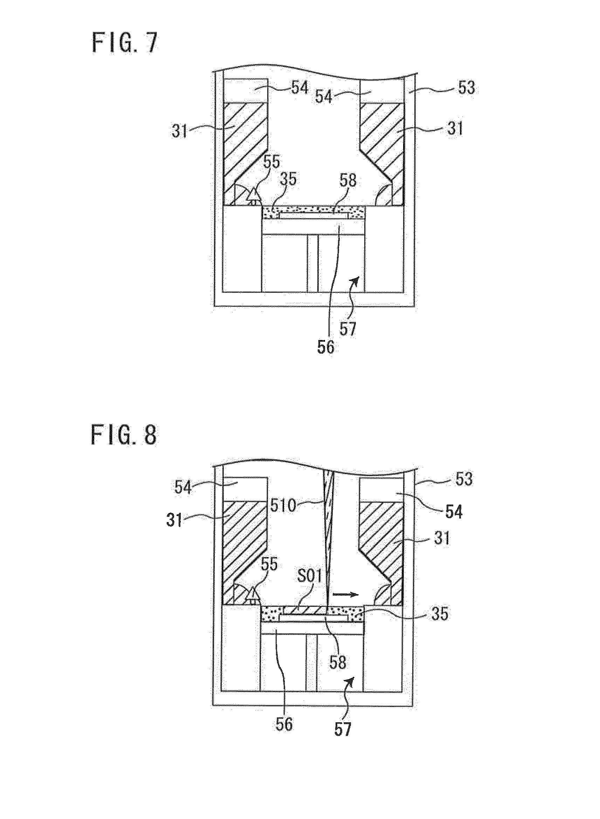

The control unit 60 forms the powder layer 35 (S6). The control unit 60 commands the pair of powder supply devices 54 to discharge a plurality of inorganic powder particles. The pair of the powder supply devices 54 discharges a plurality of inorganic powder particles in response to the command from the control unit 60. At the time, the rake 55 moves horizontally to supply the discharged inorganic powder particles to the powder storing chamber 57. As shown in Fig. 7, the inorganic powder particles are accumulated on the base plate 58 and the modeling table 56, so that the powder layer 35 is formed. The powder particles in the powder supply devices 54 do not include binder resin particles. Therefore, the powder layer 35 substantially consists of inorganic powder particles 31. The rake 55 moves further horizontally on the surface of the powder layer 35 and flattens the powder layer 35. As a result, the surface of the powder layer 35 is flattened as shown in Fig 7.

The control unit 60 then pre-heats the powder layer 35 by a well known method according to the layered manufacturing method (S7). The irradiator 51 irradiates the surface of the powder layer 35 with an electron beam 510 having a low fluence. At the time, the powder layer 35 has its temperature raised to a level in which no sintering is caused.

Then, the solidified portion in the first layer is formed by the electron beam 510 (S8). The control unit 60 reads out from the memory the processing condition data for the first layer from the plurality of pieces of processing condition data manufactured in step S2. The control unit 60 controls the electron beam 510 based on the read out processing condition data. The control unit 60 controls the regulator 52 based on the region condition in the processing condition data to irradiate a prescribed region of the powder layer 35 with the electron beam 510. The control unit 60 further controls the irradiator 51 and the regulator 52 based on the fluence condition in the processing condition data to regulate the fluence of the electron beam 510. As a result, the inorganic powder particles in the region irradiated with the electron beam 510 are melted and solidified and solidified portion SO1 in the first layer is formed on the base plate 58 as shown in Fig. 8. In the powder layer 35, inorganic powder particles 31 provided in the region other than the solidified portion SO1 are neither melted and nor sintered.

After the solidified portion SO1 in the first layer is formed, the control unit 60 determines whether the counter is nmax (S9). Here, since the counter n=1 (NO in S9), the control unit 60 increments the counter n to n+1=2 (S10). In short, the control unit 60 prepares to manufacture a solidified portion SO2 in the second layer.

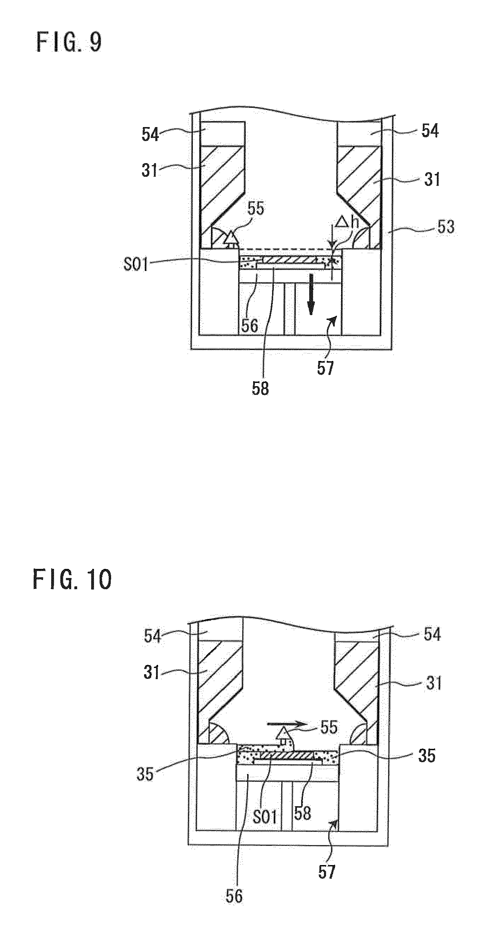

The control unit 60 lowers the modeling table 56 by a layering pitch Δh (S11). As a result, as shown in Fig. 9, the surface of the powder layer 35 is lowered by Δh as compared to Figs. 7 and 8.

After step S11, the process returns to step S6. At the time, the control unit 60 forms a new powder layer 35 on the powder layer 35 provided with the solidified portion SO1 (S6: layering step). More specifically, in response to a command from the controller unit 60, the pair of powder supply devices 54 discharges inorganic powder particles again. At the time, as shown in Fig. 10, the rake 55 moves horizontally. As a result, the inorganic powder particles are supplied to the powder storing chamber 57, so that a new powder layer 35 having a thickness of Δh is formed. The new powder layer 35 has its surface flattened by the rake 55.

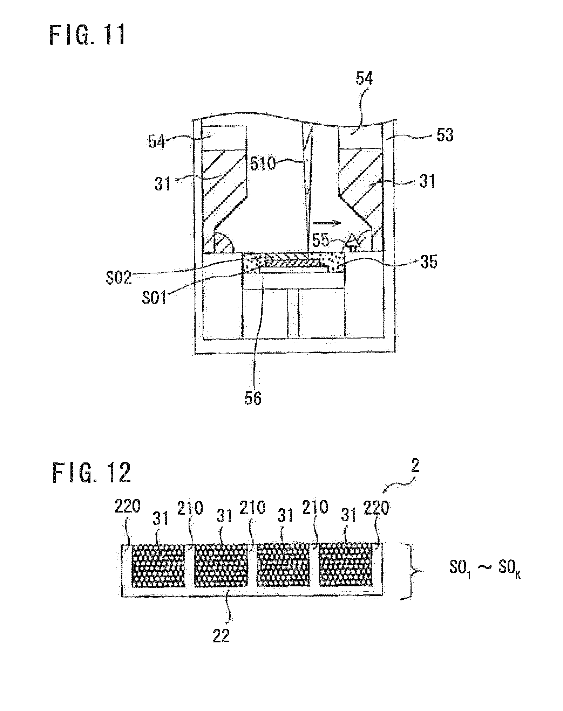

Then, the control unit 60 pre-heats the powder layer 35 (S7) and forms a solidified portion S02 in the second layer (S8: forming step). At the time, the control unit 60 irradiates the powder layer 35 with the electron beam 510 based on processing condition data for the n-th layer (n=2 in this case). As a result, referring to Fig. 11, inorganic powder particles in a region irradiated with the electron beam 510 are melted and solidified, so that a solidified portion SO2 is formed. At the time, as shown in Fig. 11, the solidified portion SO2 is layered on the solidified portion SO1.

Then, the process proceeds to step S9, and until n=nmax, in other words, until a solidified portion SOnmax in the uppermost layer is formed, the control unit 60 repeats the operation from steps S6 to S11. In short, the control unit 60 repeats the layering step (S6) and the forming step (S8) until the solidified portion 2 is completed.

Fig. 12 is a sectional view taken in the vertical direction of the solidified portion 2 in the process of manufacturing after the solidified portion SOk in the k-th layer (k is a natural number and 1<k<nmax) is formed. Referring to Fig. 12, the solidified portion 2 in the process of manufacturing is formed by the solidified portions SO1 to SOk layered on one another. The solidified portions SO1 to SOk have a plate shape, a frame shape, or a grid shape. The solidified portion 2 in the process of manufacturing has a solidified wall 22 that corresponds to the bottom wall of the solidified case 20 and a plurality of solidified walls 210 and 220 in the process of manufacturing. The solidified walls 210 correspond to the solidified walls 21 and the solidified walls 220 correspond to the solidified walls 22.

The solidified portion 2 in the process of manufacturing further stores a plurality of inorganic powder particles 31. In short, in the solidifying step, unmelted inorganic powder particles 31 remain in the solidified portion 2. The unmelted inorganic powder particles 31 stored in the solidified portion 2 are a raw material for the sintered portion 3.

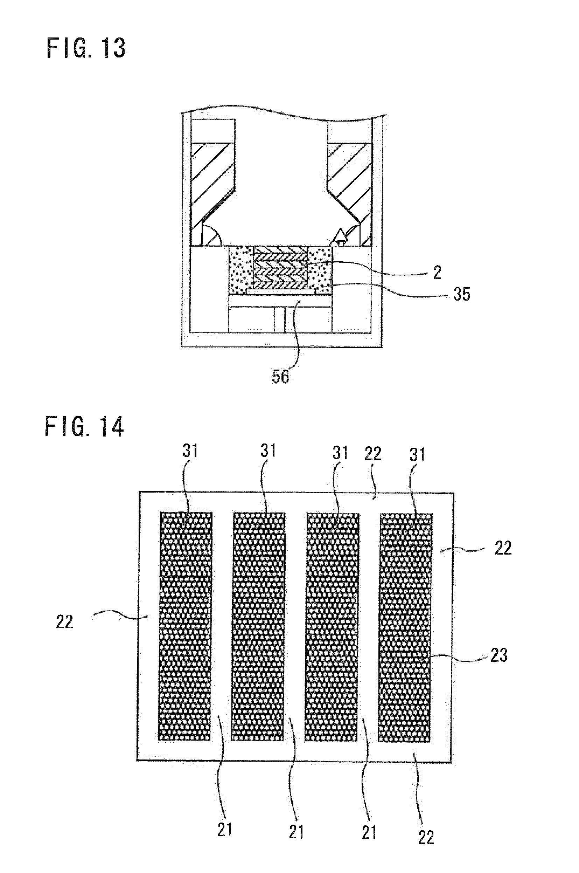

After repeatedly carrying out steps S6 to S11, when counter n=nmax, in other words, when a solidified portion SOnmax in the uppermost layer nmax is formed (YES in S9), the solidified portion 2 is completed as shown in Fig. 13. Fig. 14 is a sectional view taken in the vertical direction of the solidified portion 2 in Fig. 13. Referring to Fig. 14, the solidified portion 2 has a plurality of storing chambers 23. The storing chambers 23 store the plurality of inorganic powder particles 31. These inorganic powder particles 31 are not affected by the heat from the electron beam 510. Therefore, most of the inorganic powder particles 31 are neither melted nor sintered. Therefore, they are kept in substantially the same grain shape as the inorganic powder particles 31 discharged from the powder supply devices 54. The completed solidified portion 2 is taken out from the powder layer 35 (S12) and the manufacturing step (S100) ends.

### Sintering Step (S200)

Then, the sintering step (S200) is carried out and the sintered portion 3 is formed (S200). The solidified portion 2 taken out from the powder layer 35 is inserted in a sintering furnace. The solidified portion 2 is heated at sintering temperatures less than the melting point of the inorganic powder particles. As shown in Fig. 14, the solidified portion 2 stores a plurality of inorganic powder particles in each storing chamber 23. Therefore, the plurality of inorganic powder particles in the same storing chamber 23 are sintered and necked to one another as they are heated at the sintering temperatures, so that a plurality of necks 32 are formed. By the above-described steps, the sintered portion 3 is formed in each of the storing chambers 23. During the sintering step, the sintered portion 3 connects to each of the solidified walls 21 and 22 of the solidified portion 2.

The number and growth of the necks 32 can be controlled depending on heating time and/or heating temperatures. As the heating time prolongs, more necks 32 are formed and each of the necks 32 becomes thicker. As the heating time prolongs, the necks 32 in the sintered portions 3 become thicker and the inorganic powder particles 31 and the necks 32 are integrated into a rod or plate shape. Similarly, as the heating temperature increases, the necks 32 become thicker and the inorganic powder particles 31 and the necks 32 are integrated into a rod or a plate shape. Even in this case, a plurality of gaps 33 are formed in the sintered portion 3.

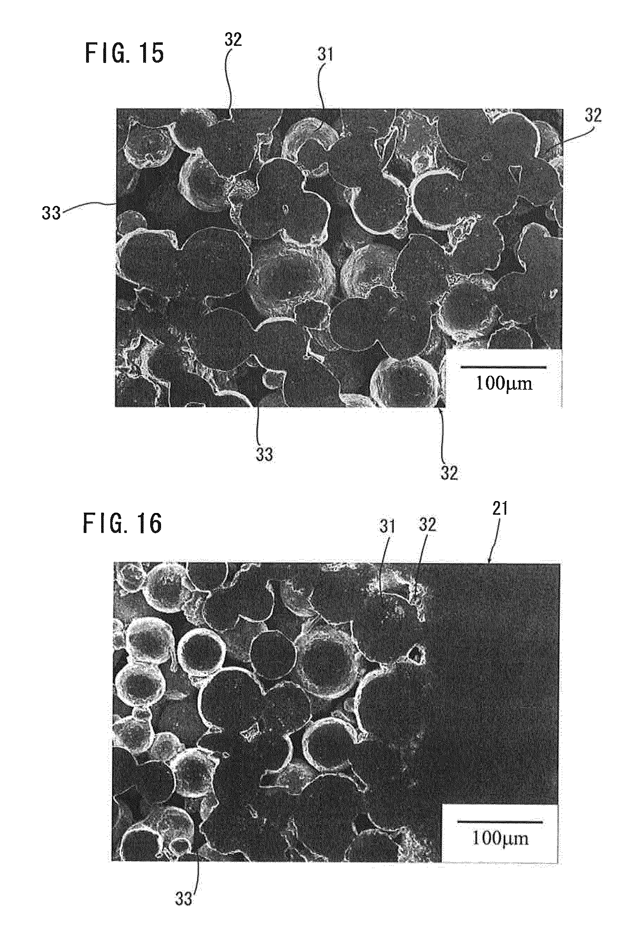

Figs. 15 and 16 show SEM images of the sintered portion 3 manufactured by the above-described method. These SEM images were obtained by the following method. Titanium 6-aluminum 4-vanadium alloy specified by JIS T7401-2:2002 was used as the inorganic powder particles 31. The grain size of the used powder particles was 45 µm to 100 µm and its average grain size was 65 µm. By the above-described manufacturing step (S100), the solidified portion 2 in the shape in Fig. 2 was formed. A plurality of inorganic powder particles 31 were stored in the formed solidified portion 2 as shown in Fig. 14.

Then, the sintering step (S200) was carried out. More specifically, the solidified portion 2 having the plurality of inorganic powder particles 31 stored therein was inserted in a sintering furnace. The solidified portion 2 was heated for 100 hours at a sintering temperature of 920°C, and a shock absorbing structure 1 was manufactured. A section of the manufactured shock absorbing structure was SEM-examined and the SEM images in Figs. 15 and 16 were obtained.

Referring to Fig. 15, the sintered portion 3 included a plurality of inorganic powder particles 31 and a plurality of necks 32. A plurality of necks 32 were formed between adjacent inorganic powder particles 31. Referring to Fig. 16, necks 32 were also formed between the solidified walls 21 and the inorganic powder particles 31. More specifically, the sintered portion 3 was connected to the solidified portion 2 by the necks 32. A plurality of gaps 33 were formed between the plurality of inorganic powder particles 31. Note that the porosity of the sintered portion 3 was 59.8%.

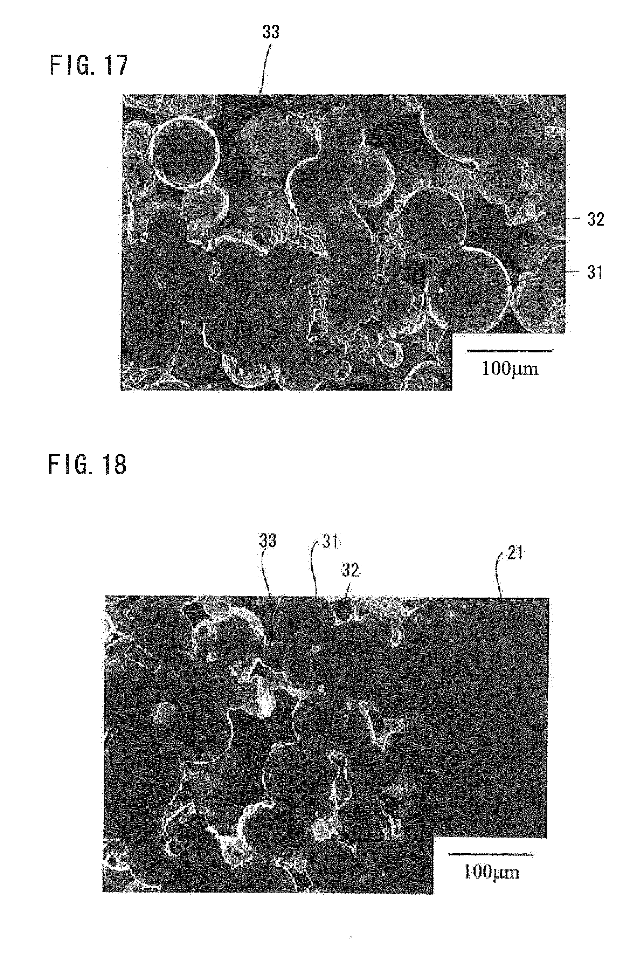

Figs. 17 and 18 show SEM images of the shock absorbing structure 1 after heated for 1000 hours in the sintering furnace. The shock absorbing structure 1 shown in Figs. 17 and 18 were manufactured under the same condition as that in Fig. 15 and 16 other than the heating time in the sintering furnace. Referring to Figs. 17 and 18, as the heating time in the sintering furnace prolonged, more necks 32 are formed and each of them were grown.

Now, characteristics of the shock absorbing structure 1 manufactured by the above-described manufacturing method will be described in detail.

### Characteristics of Shock Absorbing Structure 1

The shock absorbing structure 1 is a composite structure including the solidified portion 2 and the sintered portion 3 and has a high shock absorption characteristic. Furthermore, by the above-described manufacturing method, the Young's modulus and yield stress of the shock absorbing structure 1 can be controlled.

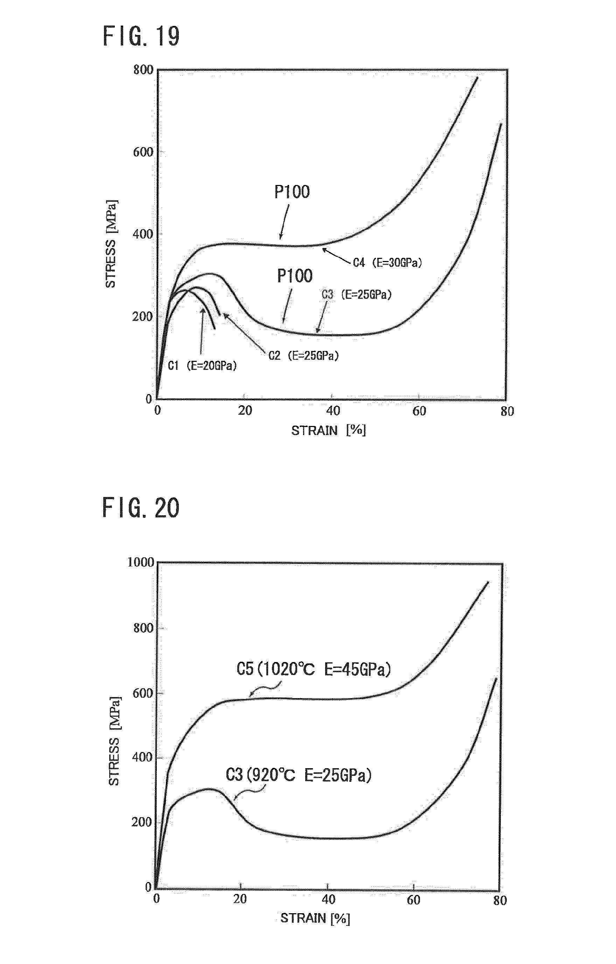

Fig. 19 is a graph showing stress-strain curves of various structures. The plurality of curves C1 to C4 shown in Fig. 19 were obtained by the following method.

Four kinds of compressed specimens shown in Table 1 were prepared.

**Table 1**

| Specimen No. | In storage chamber | Sintering temperature (°C) | Sintering time (h) |
|---|---|---|---|
| 1 | None | - | - |
| 2 | Powder particles (not sintered) | - | - |
| 3 | Sintered particles | 920 | 100 |
| 4 | Sintered particles | 920 | 1000 |

Referring to Table 1, a specimen 1 had the same structure as that of the solidified portion 2 shown in Fig. 2 and a plurality of inorganic powder particles 31 were not stored in each of the storing chambers 23. The specimen 2 had the same structure as that of the solidified portion 2 shown in Fig. 14 and a plurality of inorganic powder particles 31 were filled within each of the storing chambers 23. However, the plurality of inorganic powder particles 31 were neither melted nor sintered.

Specimens 3 and 4 had the same structure as that of the shock absorbing structure 1 and a plurality of sintered portions 3 were stored in a solidified portion 2. The specimens 3 and 4 were both manufactured by the above-described method.

Each of the specimens 1 to 4 was a cube having a size of about 10 mm × 10 mm × 10 mm. The solidified walls 21 and 22 each had a thickness from 0.4 mm to 0.6 mm, and the distance W (see Fig. 2) between adjacent solidified walls 21 and 22 was 2.5 mm.

The raw material for the solidified portion 2 and the sintered portion 3 of each of the specimens 1 to 4 was inorganic powder particles made of titanium 6-aluminum 4-vanadium alloy specified by JIST-7401-2:2002. The sintering temperature for the specimens 3 and 4 was both 920°C. However, the specimen 3 was heated for 100 hours whereas the specimen 4 was heated for 1000 hours.

Using the prepared specimens 1 to 4, compression test was carried out based on JIS H7902:2008. More specifically, compression test was carried out in the atmosphere at room temperature (25°C) using an instron type compression tester and the stress-strain curve shown in Fig. 19 was obtained. At the time, the compression direction was along the direction in which the solidified walls 21 of the specimens 1 to 4 extended (in the up-down direction in Fig. 1).

Referring to Fig. 19, the ordinate represents stress (MPa) and the abscissa represents strain (%). The curve C1 is a stress-strain curve of the specimen 1. Similarly, the curve C2 is a stress-strain curve of the specimen 2, the curve C3 is a stress-strain curve of the specimen 3, and the curve C4 is a stress-strain curve of the specimen 4. Values E at signs C1 to C4 are the Young's moduli of the specimens 1 to 4 respectively.

Referring to Fig. 19, although the specimen 1 (curve C1) and the specimen 2 (curve C2) deformed plastically, they fractured with less than 20% strain. In contrast, the specimen 3 (curve C3) and specimen 4 (C4) did not fracture even with 80% or more strain. Furthermore, the curves C3 and C4 had a plateau region P100 where the stress was substantially fixed while the strain increased.

In the plateau region P100, the stress can be kept from rising. More specifically, the specimens 3 and 4 having the plateau regions can absorb shock energy because the stress is not abruptly raised in the process of plastic deformation. Therefore, the shock absorbing structure 1 has a high shock absorption characteristic.

It is presumed that the shock absorption characteristic is obtained for the following reason. During elastic deformation, the solidified portion 2 is mainly subject to compression stress. However, after the yield point, the solidified portion 2 starts to plastically deform. At the time, a plurality of necks 32 and inorganic powder particles 31 around the necks 32 sequentially plastically deform as the strain increases. More specifically, since the solidified portion 2, the necks 32, and the inorganic powder particles 31 around the necks 32 plastically deform, the shock absorbing structure 1 continues to plastically deform without fracturing. In addition, when the necks 32 and the inorganic powder particles 31 plastically deform together with the solidified portion 2, the gaps 33 are gradually narrowed but the presence of the gaps 33 restrains rapid densification. Therefore, the plastic deformation proceeds while the stress is prevented from abruptly increasing and kept at a prescribed value. The densification of the sintered portion 3 caused by plastic deformation proceeds slowly. The plateau region is maintained until there is a level of strain large enough to substantially eliminate the gaps 33.

By the above-described mechanism, in the stress-strain curve of the shock absorbing structure 1, a plateau region with a long duration is generated, and it is presumed that the shock absorbing structure 1 has a shock absorption characteristic.

Further for the shock absorbing structure 1, by controlling the sintering temperature and the sintering time, the Young's modulus (apparent Young's modulus), the yield stress, and the shock absorbing energy of the shock absorbing structure 1 are controlled.

Referring to Fig. 19, the specimen 4 was heated for a longer period than the specimen 3 in the sintering process. Therefore, the yield stress an Young's modulus of the specimen 4 were greater than those of specimen 3. Furthermore, when the curves C3 and C4 are compared, the shock absorbing energy of the specimen 4 is greater than that of the specimen 3. It is presumed that the longer heating time caused a greater number of necks 32 to be formed and grow larger.

More specifically, based on the heating time in the sintering process, the Young's modulus, the yield stress, and the shock absorbing energy of the shock absorbing structure 1 can be controlled. As described above, if the heating time is prolonged, more necks 32 are formed and grow to be thick. Therefore, the binding between inorganic powder particles 31 in the sintering member 3 is reinforced. By controlling the number and growth of the necks 32, the Young's modulus, the yield stress, and the shock absorbing energy are controlled.

Fig. 20 is a graph of a stress-strain curve showing the effect of sintering temperatures on the shock absorbing structure 1. In the stress-strain in Fig. 20, the curve C5 was obtained by the following method. A new specimen 5 was prepared. The specimen 5 had a higher sintering temperature than that of the specimen 3. More specifically, its sintering temperature was 1020°C. The other manufacturing conditions were the same as those of the specimen 3.

When the curves C5 and C3 in Fig. 20 are compared, the yield stress of the specimen 5 is higher than that of the specimen 3. The Young's modulus of the specimen 5 obtained based on the curve C5 is 45 GPa which is higher than that of the specimen 3. Furthermore, the shock absorbing energy of the specimen 5 was greater than that of the specimen 3. It is presumed that since the sintering temperature was high, the formation and growth of necks 32 were promoted.

As described above, by controlling the sintering temperature and the heating time in the sintering process, the Young's modulus, the yield stress, and shock absorbing energy of the shock absorbing structure 1 can be controlled. More specifically, the shape of the stress-strain curve can be changed, and the period of the plateau region and the amount of shock absorbing energy corresponding to a prescribed strain amount can be controlled.

If the distance W between opposing solidified walls 21 and 22 is controlled, in other words, if the width of the storing chamber 23 is controlled, the Young's modulus, the yield stress, and the shock absorbing energy of the shock absorbing structure 1 can be controlled.

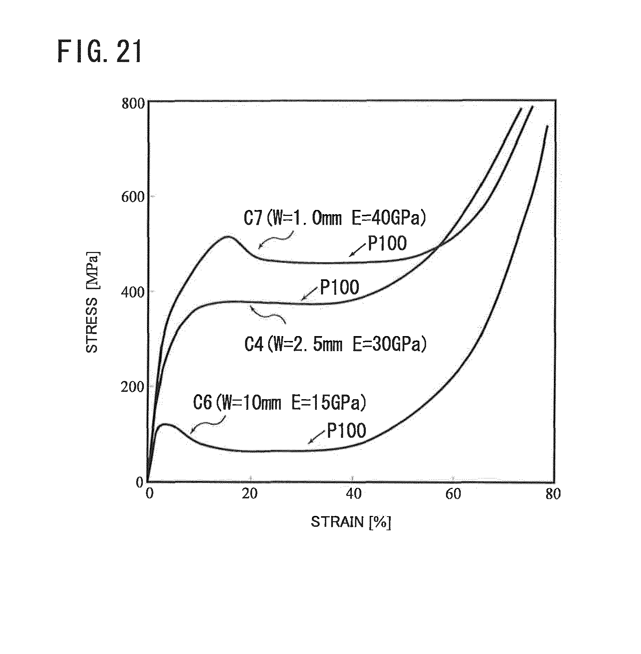

Fig. 21 is a graph including stress-strain curves of a plurality of shock absorbing structures 1 having storing chambers 23 with different widths (distances W). Curves C6 and C7 in Fig. 21 were obtained by the following method. Specimens 6 and 7 were prepared. The distance W in the specimen 6 was 10 mm which was greater than the distance W in the specimen 4 (2.5). On the other hand, the distance W in the specimen 7 was 1 mm which was smaller than the distance W in the specimen 4. The other manufacturing conditions and compression test method for the specimens 6 and 7 were the same as those for the specimen 4. Based on the obtained curves C6 and C7, the Young's moduli of the specimens 6 and 7 were obtained. The Young's modulus of the specimen 6 was 15 GPa and the Young's modulus of the specimen 7 was 40 GPa.

Referring to curves C4, C6, and C7 shown in Fig. 21, there is a plateau region P100 in each of the curves. Therefore, the specimens 4, 6, and 7 all had a shock absorption characteristic. The specimen 6 having the greater distance W than that of the specimen 4 had Young's modulus and a shock absorbing energy both smaller than those of the specimen 4. On the other hand, the specimen 7 having the smaller distance W than that of the specimen 4 had Young's modulus and a shock absorbing energy both greater than those of the specimen 4.

As in the foregoing, by controlling conditions including the sintering temperature, the heating time, and the distance W between solidified walls, the Young's modulus, the yield stress, and the shock absorbing energy of the shock absorbing structure 1 can be controlled. These conditions can be controlled by the above-described manufacturing method. Therefore, by the manufacturing method according to the embodiment, the Young's modulus, the yield stress, and the shock absorbing energy of the shock absorbing structure 1 to be manufactured can be controlled easily.

### Uses of Shock Absorbing Structure

As described above, the shock absorbing structure 1 has a stress-strain curve including a plateau region. By controlling the manufacturing conditions, its Young's modulus, yield stress, and shock absorbing energy can be controlled. Therefore, the shock absorbing structure finds various applications that require a shock absorption characteristic.

### Medical Implants

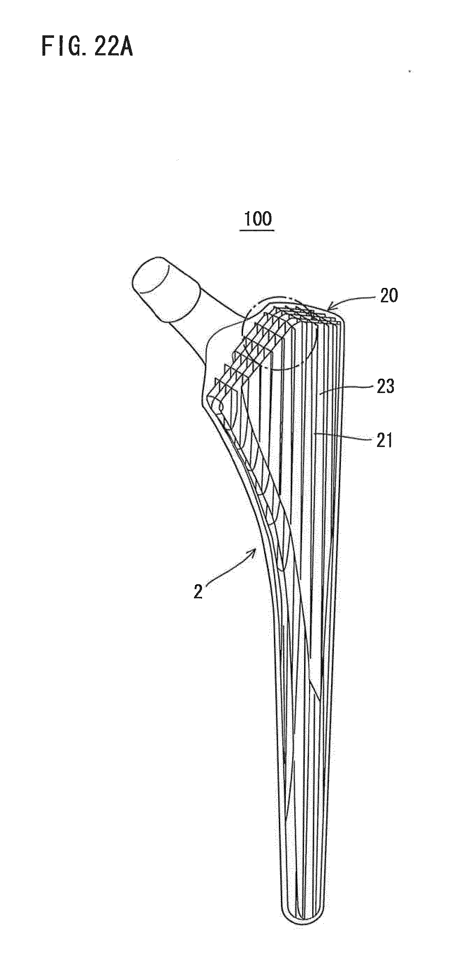

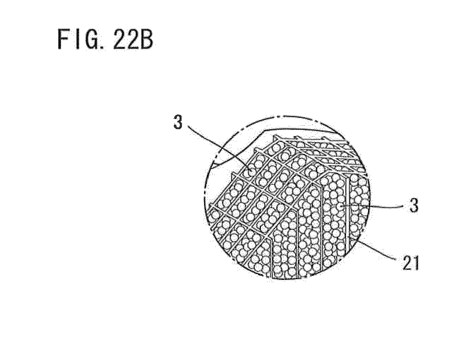

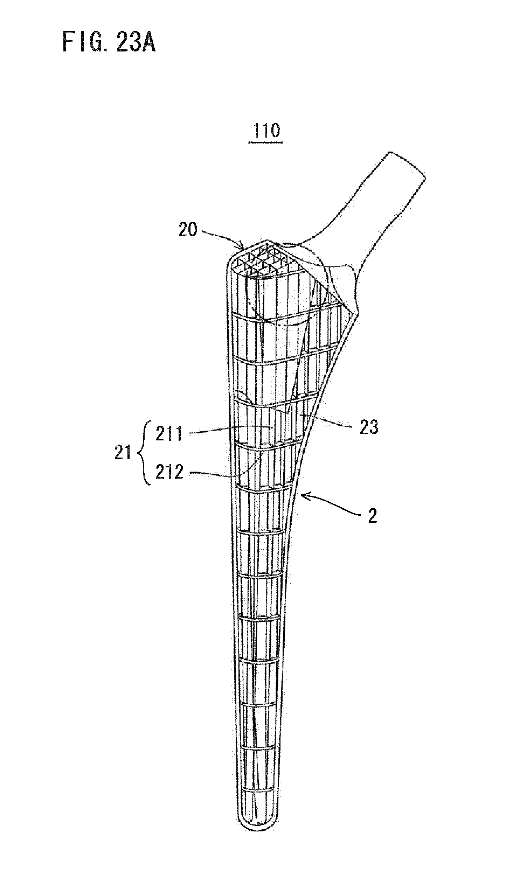

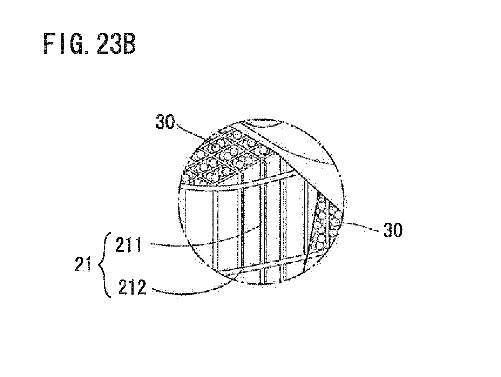

The shock absorbing structure according to the present embodiment may be used for example as a medical implant. Figs. 22A, 22B, 23A, and 23B are perspective views of a shock absorbing structure used as an artificial hip prosthesis implant. Fig. 22B is a perspective view of a region circled by a dashed line in Fig. 22A. Fig. 23B is a perspective view of the inside of a region surrounded by the dashed line in Fig. 23A. Referring to Figs. 22A, 22B, 23A, and 23B, shock absorbing structures 100 and 110 are for example inserted into a thighbone and used. The shock absorbing structures 100 and 110 each include a solidified portion 2 and a sintered portion 3 similarly to the shock absorbing structure 1. The solidified portion 2 includes a tubular solidified case 20 (that corresponds to a so-called stem portion) that has a lengthwise direction and a plurality of solidifie walls 21 provided inside the solidified case 20. The solidified walls 21 shown in Figs. 22A and 22 extend in the lengthwise direction of the solidified case 20 and arranged in the widthwise direction of the solidified case 20. An end of each of the solidified walls 21 is connected to another solidified wall 21 or the solidified case 20. The solidified walls 21 in Figs. 23A and 23B each include a first solidified wall 211 that extends in the lengthwise direction of the solidified case 20 and a second solidified wall 212 that extends in the widthwise direction of the solidified case 20 (in the horizontal direction in the figures). An end of each of the solidified walls 21 is connected to another solidified wall 21 or the solidified case 20.

The solidified case 20 shown in Figs. 22A, 22B, 23A, and 23B further has a plurality of storing chambers 23 partitioned by the plurality of solidified walls 21. The storing chambers 23 each store sintered portions 3. The sintered portions 3 are connected by a plurality of necks 32 to a plurality of solidified walls 21 provided opposed to each of the sintered portions 3.

The shock absorbing structures 100 and 110 are made of an inorganic substance similarly to the shock absorbing structure 1. The solidified portion 2 and the sintered portion 3 preferably have the same chemical composition. Inorganic powder particles 31 are preferably made of a metal. The solidified portion 2 and the sintered portion 3 are more preferably made of titanium or titanium alloy. Here, the titanium alloy is alloy containing at least 50 wt.% titanium.

The inorganic powder particles 31 that form the shock absorbing structures 100 and 110 are more preferably made of titanium or titanium alloy specified by JIS T7401. More specifically, the solidified portion 2 and the sintered portion 3 are for example made of titanium 6-aluminum 4-vanadium alloy specified by JIS T7401-2:2002 or titanium 15-zirconium 4-niobium 4-tantalu alloy specified by JIS 7041-4:2009.

The shock absorbing structures 100 and 110 are manufactured by the same manufacturing method for the shock absorbing structure 1. The solidified portion 2 is manufactured by the manufacturing step (S100), and therefore the solidified portion 2 can be manufactured into various shapes. More specifically, the solidified case 20 can be manufactured to have a desired three-dimensional shape and the plurality of solidified walls 21 in the solidified case 20 can be manufactured into a desired shape and allocated to a prescribed position.

The shock absorbing structures 100 and 110 preferably have Young's modulus in the range from 10 GPa to 50 GPa. In this case, the shock absorbing structures 100 and 110 may have Young's modulus the same or close to the Young's modulus of a bone (10 GPa to 30 GPa). Therefore, the shock absorbing structure 100 can have a mechanical characteristic analogous to a bone. As described above, using the layered manufacturing method, the thickness of the solidified wall 21 and the distance W between adjacent solidified walls 21 can be controlled in the manufacturing step (S100), so that the Young's moduli of the shock absorbing structures 100 and 110 can be controlled. Furthermore, if the sintering temperature and the heating time in the sintering step (S200) are controlled, the Young's moduli of the shock absorbing structures 100 and 110 can be controlled. Therefore, by controlling these manufacturing conditions, the Young's moduli of the shock absorbing structures 100 and 110 can be in the range from 10 GPa to 50 GPa. The Young's modulus of the shock absorbing structure is preferably from 30 GPa to 50 GPa.

As in the foregoing, the shock absorbing structure according to the present embodiment can have Young's modulus approximate to a bone. Furthermore, as shown in Figs. 19 to 21, compression stress-compression strain curves of the shock absorbing structures have a plateau region, and therefore the shock absorbing structures also have a shock absorption characteristic. Therefore, the shock absorbing structure according to the present embodiment is suitably used as a medical implant.

### Application to Transportation

The shock absorbing structure according to the present embodiment can further be used in a transportation such as an automobile, an airplane, a shop, and a train. As described above, the Young's modulus, the yield stress, and the shock absorbing energy of the shock absorbing structure 1 can be controlled as required based on manufacturing conditions in the manufacturing step (S100) and the sintering step (S200). Therefore, the shock absorbing structure has Young's modulus and yield stress depending on the kind of a moving object to be used and a stress-strain curve with a plateau region. The sintered portions in the shock absorbing structure have gaps 33, so that the shock absorbing structure is more lightweight than a solid material.

### Second Embodiment

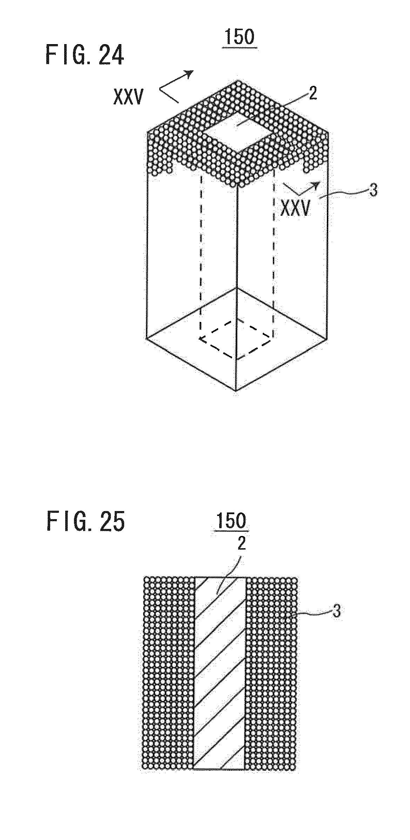

The shock absorbing structure is not limited to the structures shown in Figs. 1, 22A, and 23A. Fig. 24 is a perspective view of a shock absorbing structure 150 according to a second embodiment of the present invention. Fig. 25 is a sectional view taken along line XXV-XXV in Fig. 24. Referring to Figs. 24 and 25, the shock absorbing structure 150 includes a solidified portion 2 and a sintered portion 3 similarly to the shock absorbing structures 1 and 100. The solidified portion 2 is rod-shaped and solid. The sintered portion 3 is provided around the axis of the solidified portion 2. The sintered portion 3 is tubular and has the solidified portion 2 inserted therein. The sintered portion 3 is connected to the solidified portion 2.

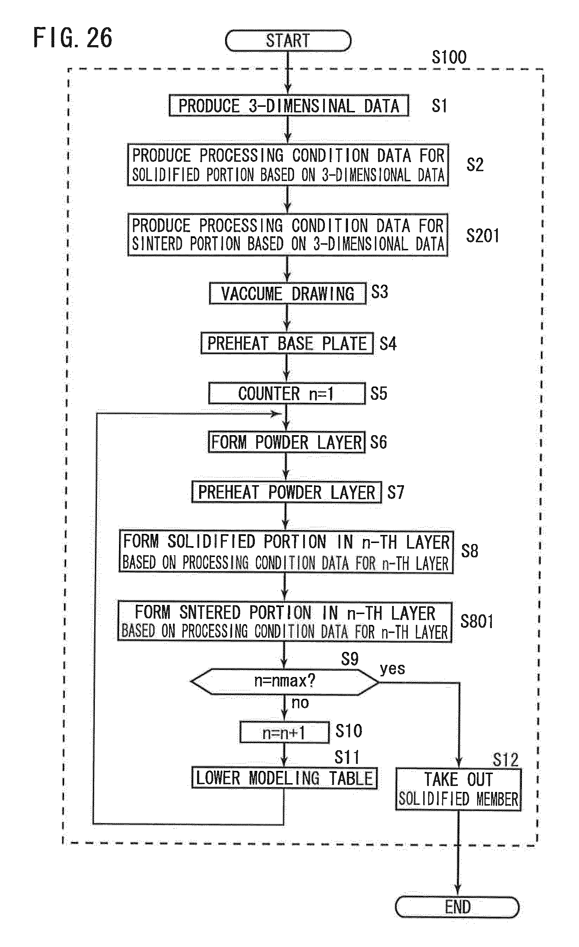

An example of a method of manufacturing the shock absorbing structure 150 will be described in the following. Fig. 26 is a flowchart showing an example of a method of manufacturing the shock absorbing structure 150. The manufacturing method shown in Fig. 26 is different from the manufacturing method in Fig. 6 in that the method additionally includes steps S201 and S801. The manufacturing method in Fig. 26 does not include the sintering step S200 shown in Fig. 6. The other steps in Fig. 26 are the same as those in Fig. 6.

The manufacturing method according to the first embodiment includes the manufacturing step (S100) and the sintering step (5200). In contrast, the manufacturing method according to the second embodiment does not include the sintering step (S200). More specifically, by the manufacturing method according to the present embodiment, the solidified portion 2 and the sintered portion 3 are manufactured in a layered manufacturing machine 50.

More specifically, the layered manufacturing machine 50 forms a plurality of solidified portions SO1 to SOnmax and a plurality of sintered portions SI1 to SInmax. The sintered portions SIn is made of the same powder layer 35 as the solidified portion SOn. When a new powder layer 35 is formed, the layered manufacturing machine 50 forms a shock absorbing portion Un including the solidified SOn and the sintered portion SIn in the new powder layer 35. The plurality of shock absorbing portions U1 to Unmax are layered and the shock absorbing structure 150 is completed. At the time, the solidified portion 2 is made of the plurality of solidified portions SO1 to SOnmax and the sintered portion 3 is made of the plurality of sintered portions SI1 to SInmax. Now, the manufacturing method according to the present embodiment will be described in detail.

Referring to Fig. 26, the control unit 60 first manufactures three-dimensional data for the shock absorbing structure 150 (S1). The manufactured three-dimensional data includes shape data for the solidified portion 2 and the sintered portion 3.

Then, the control unit 60 manufactures processing condition data for the plurality of solidified portions SOI to SOnmax that form the solidified portion 2 based on the three-dimensional data (S2). The control unit 60 manufactures processing condition data for the plurality of sintered portions SI1 to SInmax that for the sintered portion 3 (S201). The control unit 60 manufactures sectional shape data for the sintered portion 3 in the n-th layer based on the three-dimensional data. Then, the control unit 60 manufactures processing condition data based on the sectional shape data. A method of setting the processing condition data is the same as that in step S2. However, the fluence of the electron beam 510 during forming the sintered portion SIn is set smaller than the fluence of the electron beam 510 during forming the solidified portion SOn. This is for sintering the inorganic powder particles 31 without being melted.

The processing condition data for the sintered portion SIn manufactured in step S201 is stored in the memory in the control unit 60.

The control unit 60 then carries out operation in steps S3 to S5 and further forms a powder layer 35 (step S6: layering step). The control unit 60 forms a shock absorbing portion U1 in the first layer (S7, S8, and S801: forming step).

The control unit 60 pre-heats the powder layer 35 (S7). The control unit 60 then reads out the processing condition data for the solidified portion SO1 from the memory and forms the solidified SO1 (S8). The control unit 60 then reads out the processing condition data for the sintered SI1 from the memory and forms the sintered portion SI1 (S801).

The sintered portion SI1 is manufactured as follows. The control unit 60 controls the electron beam 510 based on the processing condition data. The control unit 60 controls the regulator 52 based on a region condition in the processing condition data and irradiates a prescribed region of the powder layer 35 with the electron beam 510. At the time, the control unit 60 irradiates the electron beam with lower fluence than that of the electron beam irradiated in step S8 based on the fluence condition in the processing condition data. A plurality of inorganic powder particles in the region irradiated with the electron beam 510 are heated to a temperature less than the melting point and then sintered. As a result, the sintered portion SI1 is formed. During sintering, the sintered portion SI1 is connected to the adjacent solidified SO1.

By the above-described manufacturing step, the shock absorbing portion U1 is formed in the powder layer 35. Thereafter, the control unit 60 repeats the layering step (S6) and the forming step (S7, S8, and S801) until a shock absorbing portion Unmax in the nmax-th layer is formed (S9). When the shock absorbing portion Unmax in the nmax-th layer is formed (YES in S9), the shock absorbing structure 150 is completed. The completed shock absorbing structure 150 is taken out from the powder layer 35 (S12).

Note that in Fig. 26, step S8 is carried out, followed by step S801, while step S801 may be carried out first and then step S8 may be carried out.

The shock absorbing structure 150 manufactured by the above-described manufacturing method includes a high shock absorption characteristic similarly to the shock absorbing structure 1.

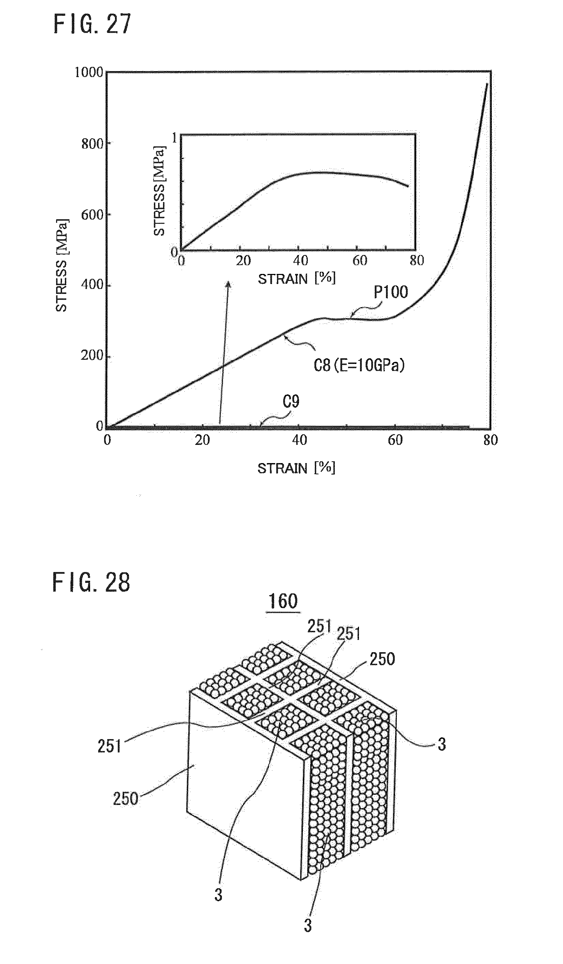

Fig. 27 is a graph of a stress-strain curve of the shock absorbing structure 150. Fig. 27 is obtained by the following method. The specimens 8 and 9 were prepared. The specimens 8 and 9 had a parallelepiped shape with a size of 5 mm × 5 × 8 mm. In the specimen 8, the solidified portion had a parallelepiped shape with a size of 1 mm × 1 mm × 8 mm and was provided in the center of the specimen 8.

The specimen 8 was manufactured by the manufacturing method shown in Fig. 26. The specimen 8 has a structure shown in Figs. 24 and 25 and corresponds to the shock absorbing structure 150. On the other hand, the specimen 9 was manufactured by compressing inorganic powder particles by cold press. Inorganic powder particles as a raw material for both the specimens 8 and 9 were titanium 6-aluminum 4-vanadium alloys specified by JIS T7401-2:2002.

The manufactured specimens 8 and 9 were subjected to compression tests by the same method as that carried out to the specimens 1 to 7 and stress-strain curves shown in Fig. 27 were obtained.

Referring to Fig. 27, the curve C8 is a stress-strain curve of the specimen 8 and the curve C9 is a stress-strain curve of the specimen 9. The Young's modulus of the specimen 8 is shown in Fig. 27. The specimen 9 yielded to very low stress because the specimen 9 was formed simply by compressing the inorganic powder particles and did not show a shock absorption characteristic. On the other hand, Young's modulus E and yield stress of the specimen 8 were both higher than those of the specimen 9. Note that the Young's modulus was 10 GPa that was approximate to the Young's modulus of a bone. Furthermore, the curve C8 has a plateau region P100. Therefore, by carrying out step S801 to form the solidified portion 3, the low Young's modulus and a shock absorption characteristic were obtained.

As in the foregoing, the shock absorbing structure 150 has a shock absorption characteristic similarly to the shock absorbing structure 1. The shock absorbing structure 150 that is lightweight and may have low Young's modulus is suitably applied to a medical implant.

The shapes of the shock absorbing structures according to the first and second embodiments are not limited to those shown in Fig. 1, and 22A to 25. Fig. 28 shows another example of the shock absorbing structure. Referring to Fig. 28, the solidified portion 2 of the shock absorbing structure 160 includes two solidified walls 250 provided opposed to each other and a plurality of solidified walls 251 provided between the two solidified walls 250. A plurality of solidified portions 3 are stored in the solidified walls 250 and 251. The shock absorbing structure 160 having the above-described shape can be manufactured by the method shown in Fig. 26.

ternatively, the solidified portion 2 of the shock absorbing structure may be rod-shaped or made of a single plate shown in Figs. 24 and 25. The solidified portion 2 may have a frame shape or a grid shape including a combination of a plurality of rods. In short, the shock absorbing structure according to the present invention needs only include a solidified portion 2 having a shape not particularly specified and a sintered portion 3 connected to the solidified portion 2. These shock absorbing structures can be manufactured by the manufacturing method shown in Fig. 26.

Note that the shock absorbing structures 1, 100 and 110 can also be manufactured by the manufacturing method shown in Fig. 26.

As shown in Figs. 1, 22A, and 23A, when the solidified portion 2 includes the solidified case 20, the shape of the solidified case 20 is not particularly limited. The solidified case 20 may be parallelepiped as shown in Fig. 1 or have a curved surface as shown in Figs. 22A and 23A. The solidified case 20 is formed by the layered manufacturing method and therefore the shape is not particularly limited.

The solidified case 20 does not have to be completely sealed. For example, one or more through holes may be formed at a solidified wall 22 that corresponds to an outer wall of the solidified case 20. A through hole may be formed at a solidified wall 21 that corresponds to an inner wall of the solidified case 20. Each of the solidified walls 21 and 22 may be in a grid shape including a combination of a plurality of rods.

By the manufacturing methods according to the first and second embodiments, the inorganic powder particles 31 are melted by the electron beam 510 to manufacture the solidified portion 2. However, instead of the electron beam 510, the inorganic powder particles 31 may be melted by a laser beam for example from a CO2 laser, a YAG layer, or a semiconductor laser. In short, the inorganic powder particles 31 are melted by a beam and the solidified portion 2 is formed.

By the manufacturing method according to the second embodiment (Fig. 26), step S801 may be carried out before step S8 or step S8 may be carried out after step S801. More specifically, a sintered portion may be formed first and then a solidified portion may be formed. In this case, the sintered portion 3 is connected to the solidified portion 2 as the sintered portion 3 and/or the solidified portion 2 are partly melted.

The plurality of inorganic powder particles 31 used according to the first and second embodiments may include different kinds of inorganic powder particles with different chemical compositions or may have the same chemical composition among them.

Although the embodiments of the present invention have been described, the same is by way of illustration and example only. Therefore, the present invention is not limited by the above-described embodiments and the above-described embodiments are susceptible to variations and modifications without departing the scope and spirit of the present invention.

### INDUSTRIAL APPLICABILITY

The shock absorbing structure according to the present invention is applicable to a field that needs a shock absorbing characteristic. It can be particularly advantageously used in a transportation such as an automobile, an airplane, a ship, and a train, and a medical implant.

## Claims

1. A shock absorbing structure, comprising:
a solidified portion formed by dissolving a plurality of inorganic powder particles; and
a sintered portion formed by sintering a plurality of said inorganic powder particles and connected to said solidified portion.

2. The shock absorbing structure according to claim 1, wherein said sintered portion comprises:
a plurality of necks formed between a plurality of said inorganic powder particles; and
a gap formed between the plurality of said inorganic powder particles.

3. The shock absorbing structure according to claim 2, wherein said solidified portion comprises a solidified case, and
said sintered portion is stored in and connected to said solidified case.

4. The shock absorbing structure according to claim 3, wherein said solidified portion further comprises:
a solidified wall formed in said solidified case; and
a plurality of storing chambers provided in said solidified case and partitioned by said solidified wall, and
said shock absorbing structure comprises a plurality of said sintered portions stored in said storing chambers and connected to said solidified case and/or said solidified wall.

5. The shock absorbing structure according to claim 3, wherein a plurality of solidified portions are sequentially layered by an layered manufacturing method, so that said solidified portion that stores a plurality of said inorganic powder particles is formed, and
said solidified portion thus formed is heated at a sintering temperature less than a melting point of said inorganic powder particles, so that said sintered portion is formed.

6. The shock absorbing structure according to claim 3, wherein a plurality of shock absorbing portions are sequentially layered by an layered manufacturing method,
each said shock absorbing portion comprises a solidified portion formed by irradiating a powder layer made of a plurality of said inorganic powder particles with a first beam, thereby dissolving a first region of said powder layer; and
a sintered portion formed by irradiating said powder layer with a second beam having a fluence lower than that of said first beam, thereby sintering a second region of said powder layer different from the first region.

7. The shock absorbing structure according to claim 1, wherein said inorganic powder particles are made of a metal.

8. The shock absorbing structure according to claim 7, wherein said solidified portion has the same composition as that of said sintered portion.

9. The shock absorbing structure according to claim 8, wherein said solidified portion and said sintered portion are made of titanium alloy.

10. The shock absorbing structure according to claim 9, having Young's modulus from 10 GPa to 50 GPa.

11. A method of manufacturing a shock absorbing structure comprising a solidified portion formed by dissolving a plurality of inorganic powder particles and a sintered portion formed by sintering a plurality of said inorganic powder particles, comprising the steps of:
forming a powder layer made of a plurality of said inorganic powder particles;
forming a solidified portion by irradiating a prescribed region of said powder layer with a beam, and dissolving said inorganic powder particles;
layering a new powder layer made of said plurality of inorganic powder particles on said powder layer provided with said solidified portion;
forming a new solidified portion by irradiating a prescribed region of said new powder layer with a beam;
forming a solidified portion made of the plurality of said layered solidified portions and storing a plurality of the inorganic powder particles by repeating said layering step and said forming step;
taking out said solidified portion from said powder layer; and
forming said sintered portion by heating said taken out solidified portion at a sintering temperature less than a melting point of said inorganic powder particles.

12. A method of manufacturing a shock absorbing structure comprising a solidified portion formed by dissolving a plurality of inorganic powder particles and a sintered portion formed by sintering a plurality of said inorganic powder particles, comprising the steps of:
forming a powder layer made of a plurality of inorganic powder particles;
forming a solidified portion by irradiating a first region of said powder layer with a first beam and dissolving a plurality of said powder particles;
forming a sintered portion by irradiating a second region of said powder layer different from said first region with a second beam with a fluence lower than that of said first beam and sintering a plurality of said inorganic powder particles;
layering a new powder layer on the powder layer provided with said solidified portion and said sintered portion;
forming said solidified portion and said sintered portion with said new powder layer; and
forming said shock absorbing structure comprising said solidified portion made of a plurality of layered solidified portions and said sintered portion made of a plurality of layered sintered portions by repeating said layering step and said forming steps.
